# EUROPÄISCHE PATENTSCHRIFT

(11) **EP 1 495 737 B1**
(45) Veröffentlichungstag und Bekanntmachung des Hinweises auf die Patenterteilung: **23.04.2008**
(21) Anmeldenummer: 04014424.8
(22) Anmeldetag: 19.06.2004
(51) Int. Cl.: A61F 2/18

(54) **Gehörknöchelchenprothese mit integriertem Kugelgelenk**
Auditory ossicles prosthesis with integrated ball joint
Prothèse d'osselet de l'ouie à rotule integrée

(30) Priorität: 10.07.2003 DE 20310609 U
(43) Veröffentlichungstag der Anmeldung: 12.01.2005
(73) Patentinhaber: Heinz Kurz GmbH Medizintechnik, 72144 Dusslingen (DE)
(72) Erfinder: Steinhardt, Uwe, 72145 Hirrlingen (DE); àWengen, Daniel F., Dr., 4102 Binningen (CH)
(74) Vertreter: Kohler Schmid Möbus

(56) Entgegenhaltungen:
- DE-U1- 20 014 659
- DE-U1- 20 212 771
- FR-A- 2 675 372
- US-B1- 6 540 661

## Beschreibung

Die Erfindung betrifft eine Gehörknöchelchenprothese, die mit ihrem einen Ende am Hammergriff der menschlichen Gehörknöchelchenkette befestigt, und die mit ihrem anderen Ende am Steigbügel der menschlichen Gehörknöchelchenkette befestigt oder direkt ins Innenohr getaucht wird.

Gehörknöchelchenprothesen werden verwendet, um bei ganz oder teilweise fehlendem oder beschädigtem Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise am Hammergriff der menschlichen Gehörknöchelchenkette befestigt, und das andere Ende der Gehörknöchelchenprothese beispielsweise am Steigbügel der menschlichen Gehörknöchelchenkette befestigt oder direkt ins Innenohr getaucht wird.

Da die anatomischen Gegebenheiten des Ohres, wie beispielsweise die Lage, die Form und die Größe des Steigbügels, des Hammers und des Trommelfells variieren, und da es sein kann, dass auch nach einer Operation, bei der die Gehörknöchelchenprothese eingesetzt wurde, noch Veränderungen in der Knochen-und Knorpelstruktur auftreten können, ist es vorteilhaft, wenn die Gehörknöchelchenprothese nicht starr ausgebildet ist, sondern eine gewisse Flexibilität aufweist. Um diese Flexibilität zu erreichen, sind verschiedene Befestigungsvorrichtungen und Ankopplungsvorrichtungen für Gehörknöchelchen, die elastische Teile und/oder Gelenke aufweisen, vorgeschlagen worden.

Der Erfindung liegt die Aufgabe zugrunde, eine Gehörknöchelchenprothese anzugeben, bei der keine getrennten Befestigungsvorrichtungen oder Ankoppelungsvorrichtungen vorgesehen werden müssen, um die geforderte Flexibilität zu erzielen, sondern die als Prothese selbst diese Flexibilität aufweist.

Die gestellte Aufgabe wird erfindungsgemäß durch die Gehörknöchelchenprothese, die die im Hauptanspruch aufgeführten Merkmale aufweist, gelöst. Die Unteransprüche geben bevorzugte Weiterbildungen an.

Eine Gehörknöchelchenprothese aus DE-U-20014659 weist selbst mindestens ein integriertes Gelenk auf, um die geforderte Flexibilität zu erzielen. Dadurch ist die Gehörknöchelchenprothese selber in sich beweglich und benötigt keine speziellen Befestigungs- und Ankoppelungsvorrichtungen, um die geforderte Flexibilität oder Beweglichkeit zu gewährleisten.

Bei der erfindungsgemäßen Gehörknöchelchenprothese handelt es sich bei dem Gelenk um ein in mehreren Richtungen bewegliches Kugelgelenk. Es werden mehrere Kugelgelenke, beispielsweise drei Kugelgelenke, hintereinander angeordnet, wobei ein nahezu beliebiger Bewegungsspielraum der Gehörknöchelchenprothese, der sowohl die Richtung als auch die Längenerstreckung umfasst, erzielt werden kann.

Das Kugelgelenk kann beispielsweise aus einer an einem Stab befestigten Kugel, die in einem U-förmigen Pfannenteil, das zum besseren Halten der Kugel in den längeren Seitenwänden auch noch Öffnungen aufweisen kann, ausgebildet werden.

Die Gehörknöchelchenprothese muss an ihren beiden Enden befestigt werden. Beispielsweise erfolgt die Befestigung des einen Endes am Hammergriff der menschlichen Gehörknöchelchenkette und die Befestigung des anderen Endes am Steigbügel der menschlichen Gehörknöchelchenkette oder direkt im Innenohr. In einer vorteilhaften Ausführungsform ist zur Befestigung der erfindungsgemäßen Gehörknöchelchenprothese am Hammergriff bzw. am Steigbügel an ihrem jeweiligen Ende jeweils ein Clip vorgesehen, der zur Befestigung über den Hammergriff bzw. den Steigbügel geschoben wird. Der Clip kann zwei V- oder U-förmig angeordnete Federzungen für das Einklicken aufweisen. Damit der Halt des Clips verbessert wird, sind in einer vorteilhaften Ausführungsform die Kontaktstellen des Clips zum Hammergriff bzw. zum Steigbügel aufgeraut. Damit das Befestigen des Clips beim Einsetzen der Prothese erleichtert wird, kann der Clip einen Haltegriff beispielsweise in Form eines Verlängerungssteges aufweisen.

Zur Befestigung der Gehörknöchelchenprothese im Innenohr wird vorteilhafterweise statt des Clips ein Kolben verwendet.

Alle Komponenten der Gehörknöchelchenprothese müssen, um Abstoßungsreaktionen zu vermeiden, aus einem biokompatiblen Material hergestellt sein. Als Materialien kommen beispielsweise Titan, Titanlegierungen oder Nitinol (NiTi) in Frage.

Zwei Ausführungsformen einer ausgebildeten Gehörknöchelchenprothese werden nachfolgend anhand der Zeichnungen erläutert.

In den Zeichnungen sind gleiche Elemente in allen Zeichnungsfiguren mit den gleichen Bezugszahlen gekennzeichnet.

Es zeigen:
- Fig. 1: eine perspektivische Darstellung einer Ausführungsform einer ausgebildeten Gehörknöchelchenprothese nach dem Stand der Technik; und
- Fig. 2: eine perspektivische Darstellung einer Ausführungsform einer erfindungsgemäß ausgebildeten Gehörknöchelchenprothese.

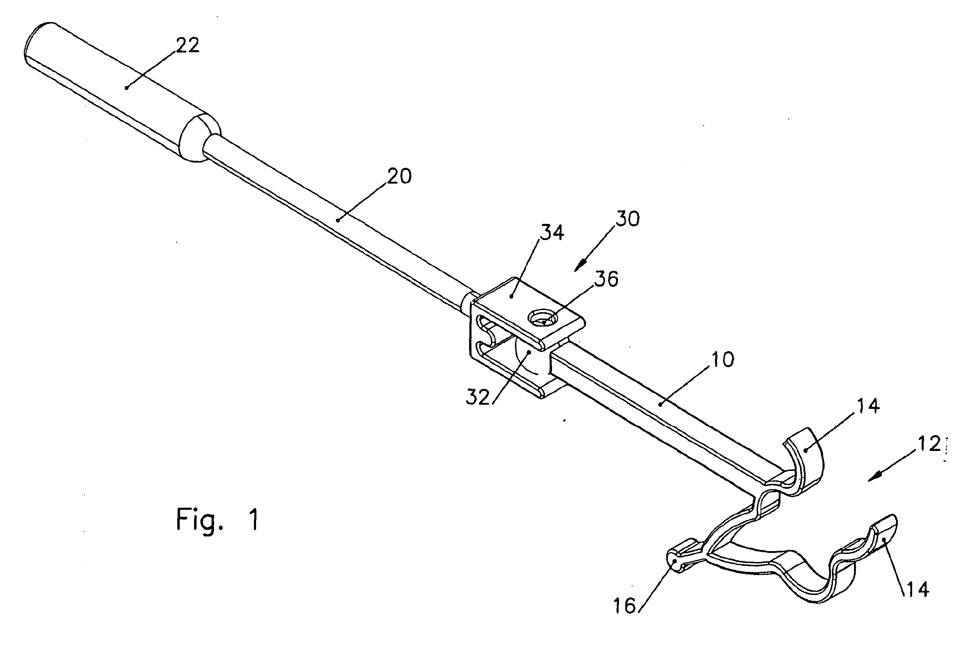

Fig. 1 zeigt in perspektivischer Darstellung eine Ausführungsform einer ausgebildeten Gehörknöchelchenprothese. Die Gehörknöchelchenprothese besteht in dieser Ausführungsform aus zwei Teilen, nämlich einem ersten Stab 10 und einem zweiten Stab 20, die über ein Gelenk 30 beweglich miteinander verbunden werden.

Der erste Stab 10 weist an seinem einen Ende einen ersten Clip 12 auf, mit dem er an einem hier nicht dargestellten Hammergriff der menschlichen Gehörknöchelchenkette befestigt werden kann. Der erste Clip 12 weist in dieser Ausführungsform zwei bewegliche, V-förmige Federzungen 14 auf, mit denen er über den Hammergriff geschoben werden kann. Ein Ende des ersten Clips 12 ist in Form eines Stegs als Haltegriff 16 ausgebildet, so dass der Clip 12 beim Einsetzen in das Ohr an diesem Haltegriff 16 leicht gehalten werden kann.

An seinem entgegengesetzten Ende weist der erste Stab 10 eine Kugel 32 auf. Sie dient als Kugel in einem hier zur Verbindung der beiden Stäbe benutzten Kugelgelenk. Die Kugel ist in einer Pfanne 34 gelagert. Die Pfanne 34 ist mit dem zweiten Stab 20 verbunden. In der hier dargestellten Ausführungsform ist die Pfanne 34 U-förmig ausgebildet und weist an ihren längeren Seitenwänden zwei Öffnungen 36 zur definierten Lagerung der Kugel 32 auf.

Der zweite Stab 20 weist an seinem entgegengesetzten Ende in dieser Ausführungsform einen Kolben 22 auf. Mit diesem Kolben kann er direkt im Innenohr befestigt werden.

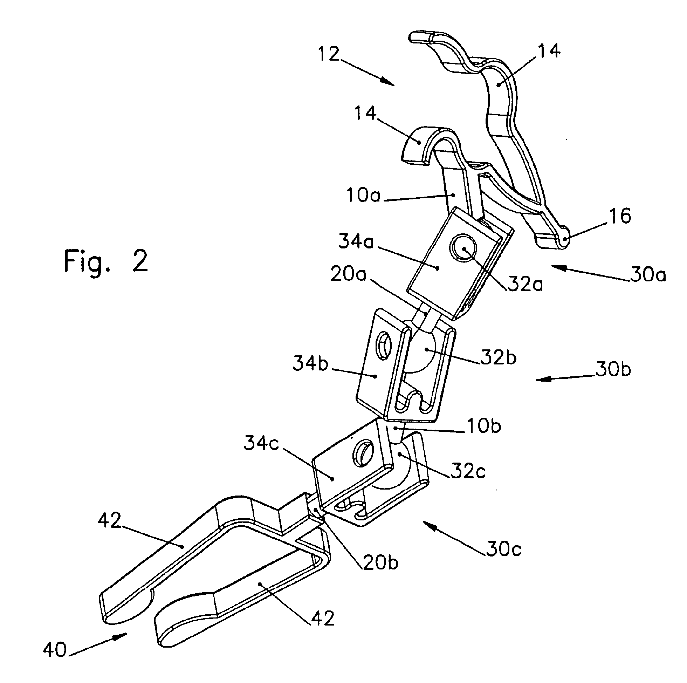

Fig. 2 zeigt in perspektivischer Darstellung eine Ausführungsform einer erfindungsgemäß ausgebildeten Gehörknöchelchenprothese. Bei dieser Ausführungsform der Gehörknöchelchenprothese sind die ersten und zweiten Stäbe sehr verkürzt, da sie dieses Mal über drei hintereinander angeordnete Gelenke 30a, 30b , 30c miteinander verbunden sind, und somit als zwei erste Stäbe 10a, 10b und zwei zweite Stäbe 20a, 20b ausgebildet sind. Diese Verbindung über mehrere hintereinander angeordnete Gelenke ermöglicht eine flexiblere richtungs- und längenmäßige Verstellung der Gehörknöchelchenprothese, aber die Stäbe müssen entsprechend verkürzt werden, damit die Prothese noch in das Innenohr hineinpasst. Die Gelenke 30a, 30b, 30c sind wieder mit Kugel 32a, 32b und 32c und Pfanne 34a, 34b, 34c so aufgebaut wie das Gelenk in der in Fig. 1 dargestellten Ausführungsform.

Die Gehörknöchelchenprothese weist in dieser Ausführungsform an ihrem einen Ende wieder den ersten Clip 12 zur Befestigung am Hammergriff der menschlichen Gehörknöchelchenkette auf. An ihrem anderen Ende weist sie einen zweiten Clip 40, der dieses Mal zur Befestigung am Steigbügel der menschlichen Gehörknöchelchenkette vorgesehen ist, auf. Er besteht in dieser Ausführungsform aus zwei U-förmig miteinander verbundenen Federzungen 42.

### Bezugszeichenliste:

- 10, 10a, 10b: erster Stab
- 12: erster Clip
- 14: Federzunge
- 16: Haltegriff

- 20, 20a, 20b: zweiter Stab
- 22: Kolben

- 30, 30a, 30b, 30c: Gelenk
- 32, 32a, 32b, 32c: Kugel
- 34, 34a, 34b, 34c: Pfanne
- 36: Öffnung

- 40: zweiter Clip
- 42: Federzunge

## Patentansprüche

1. Gehörknöchelchenprothese, die an einem Ende am Hammergriff der menschlichen Gehörknöchelchenkette, am anderen Ende am Steigbügel der menschlichen Gehörknöchelchenkette befestigbar ist oder direkt ins Innenohr getaucht werden kann und die mindest ein integriertes Gelenk (30) umfasst, **dadurch gekennzeichnet, dass** mehrere hintereinander angeordnete Kugelgelenke (30a, 30b, 30c) vorgesehen sind.

2. Gehörknöchelchenprothese nach Anspruch 1, **dadurch gekennzeichnet, dass** das Gelenk (30) aus einem in mehreren Richtungen beweglichen Kugelgelenk besteht.

3. Gehörknöchelchenprothese nach Anspruch 1 oder 2, **dadurch gekennzeichnet, dass** das Kugelgelenk aus einer an einem ersten Stab (10) befestigten Kugel (32), die in einem U-förmigen Pfannenteil (34) gelagert ist, besteht.

4. Gehörknöchelchenprothese nach Anspruch 3, **dadurch gekennzeichnet, dass** der U-förmige Pfannenteil (34) in den längeren Seitenwänden Öffnungen (36) aufweist, in denen die Kugel (32) gelagert ist.

5. Gehörknöchelchenprothese nach einem der vorhergehenden Ansprüche, **dadurch gekennzeichnet, dass** drei hintereinander angeordnete Kugelgelenke (30a, 30b, 30c) vorgesehen sind.

6. Gehörknöchelchenprothese nach einem der vorhergehenden Ansprüche, **dadurch gekennzeichnet, dass** sie zur Befestigung am Hammergriff wie auch zur Befestigung am Steigbügel einen Clip (12, 40) aufweist.

7. Gehörknöchelchenprothese nach Anspruch 6, **dadurch gekennzeichnet, dass** der Clip (12, 40) zwei V- oder U-förmig angeordnete Federzungen (14, 42) aufweist.

8. Gehörknöchelchenprothese nach Anspruch 6 oder 7, **dadurch gekennzeichnet, dass** der Clip (12, 40) an seinen Kontaktstellen zum Hammergriff bzw. zum Steigbügel aufgeraut ist.

9. Gehörknöchelchenprothese nach einem der Ansprüche 6 bis 8, **dadurch gekennzeichnet, dass** der Clip (12, 40) einen Haltegriff (16) aufweist.

10. Gehörknöchelchenprothese nach Anspruch 6, **dadurch gekennzeichnet, dass** sie zur Befestigung direkt im Innenohr statt des Clips einen Kolben (22) aufweist.

11. Gehörknöchelchenprothese nach einem der vorhergehenden Ansprüche, **dadurch gekennzeichnet, dass** alle ihre Komponenten aus Titan, einer Titanlegierung oder aus Nitinol (NiTi) hergestellt sind.

## Claims

1. Auditory ossicles prosthesis which can be attached at one end to the handle of the hammer and at its other end to the stirrup of the human auditory ossicles chain or directly introduced into the internal ear and which includes at least one integrated joint (30), **characterised in that** a plurality of ball and socket joints (30a, 30b, 30c) are provided arranged after one another.

2. Auditory ossicles prosthesis according to claim 1, **characterised in that** the joint (30) consists of a ball and socket joint movable in a plurality of directions.

3. Auditory ossicles prosthesis according to claim 1 or 2, **characterised in that** the ball and socket joint consists of a ball (32) which is attached to a first rod (10) and carried in a U-shaped socket member (34).

4. Auditory ossicles prosthesis according to claim 3, **characterised in that** the U-shaped socket member (34) has openings (36) in its longer side walls in which the ball (32) is carried.

5. Auditory ossicles prosthesis according to one of the preceding claims, **characterised in that** three ball and socket joints (30a, 30b, 30c) are provided arranged after one another.

6. Auditory ossicles prosthesis according to one of the preceding claims, **characterised in that**, for attachment to the handle of the hammer and also for attachment to the stirrup, it has a clip (12, 40).

7. Auditory ossicles prosthesis according to claim 6, **characterised in that** the clip (12, 40) has two spring tongues (14, 42) arranged to be V- or U-shaped.

8. Auditory ossicles prosthesis according to claim 6 or 7, **characterised in that** the clip (12, 40) is roughened at its contact points with the handle of the hammer, respectively with the stirrup.

9. Auditory ossicles prosthesis according to one of claims 6 to 8, **characterised in that** the clip (12, 40) has a grip handle.

10. Auditory ossicles prosthesis according to claim 6, **characterised in that**, for attachment directly in the internal ear, it has a piston (22) instead of the clip.

11. Auditory ossicles prosthesis according to one of the preceding claims, **characterised in that** all its components are made of titanium, a titanium alloy or of Nitinol (NiTi).

## Revendications

1. Prothèse de l'osselet de l'oreille qui est fixée à une extrémité du manche du marteau de la chaîne ossiculaire humaine, à l'autre extrémité de l'étrier de la chaîne ossiculaire humaine ou qui peut être plongée directement dans l'oreille interne et qui comprend au moins une articulation intégrée (30), **caractérisée en ce que** plusieurs articulations à rotules disposées les unes derrière les autres (30a, 30b, 30c) sont prévues.

2. Prothèse de l'osselet de l'oreille selon la revendication 1, **caractérisée en ce que** l'articulation (30) est composée d'une articulation à rotule mobile dans plusieurs directions.

3. Prothèse de l'osselet de l'oreille selon la revendication 1 ou 2, **caractérisée en ce que** l'articulation à rotule est composée d'une rotule (32) fixée à une première tige (10) et qui est logée dans une partie de cupule en U (34).

4. Prothèse de l'osselet de l'oreille selon la revendication 3, **caractérisée en ce que** la partie de cupule en U (34) comprend des ouvertures (36) dans de longues parois latérales, dans lesquelles la rotule (32) est logée.

5. Prothèse de l'osselet de l'oreille selon l'une des revendications précédentes, **caractérisée en ce que** trois articulations à rotules (30a, 30b, 30c) disposées les unes derrière les autres sont prévues.

6. Prothèse de l'osselet de l'oreille selon l'une des revendications précédentes, **caractérisée en ce qu'**elle comprend un clip (12, 40) pour une fixation sur le manche du marteau aussi bien que pour une fixation sur l'étrier.

7. Prothèse de l'osselet de l'oreille selon la revendication 6, **caractérisée en ce que** le clip (12, 40) comprend deux languettes flexibles disposées en forme de V ou de U (14, 42).

8. Prothèse de l'osselet de l'oreille selon la revendication 6 ou 7, **caractérisée en ce que** le clip (12, 40) est gratté au niveau de son point de contact au manche du marteau et/ou à l'étrier.

9. Prothèse de l'osselet de l'oreille selon les revendications 5 à 8, **caractérisée en ce que** le clip (12, 40) comprend une poignée (16).

10. Prothèse de l'osselet de l'oreille selon la revendication 6, **caractérisée en ce qu'**elle comprend un piston (22) pour une fixation directement dans l'oreille interne à la place du clip.

11. Prothèse de l'osselet de l'oreille selon l'une des revendications précédentes, **caractérisée en ce que** tous ses composants sont fabriqués à base de titane, d'un alliage de titane ou de Nitinol (NiTi).
